# FASCICULE DE BREVET EUROPEEN

(11) **EP 1 395 825 B1**
(45) Date de publication et mention de la délivrance du brevet: **11.04.2007**
(21) Numéro de dépôt: 02745526.0
(22) Date de dépôt: 13.06.2002
(51) Int. Cl.: G01N 33/564, G01N 33/68

(54) **PROCEDE DE DETECTION D'AUTO-ANTICORPS SPECIFIQUES DE LA POLYARTHRITE RHUMATOIDE**
VERFAHREN ZUM NACHWEIS VON RHEUMATOIDEN POLYARTHRITIS-SPEZIFISCHEN AUTOANTIKÖRPERN
METHOD FOR DETECTING RHEUMATOID ARTHRITIS-SPECIFIC AUTOANTIBODIES

(30) Priorité: 13.06.2001 FR 0108068
(43) Date de publication de la demande: 10.03.2004
(73) Titulaire: BIOMERIEUX, 69280 Marcy-L'Etoile (FR)
(72) Inventeur: INCAURGARAT, Brigitte, F-69210 Lentilly (FR); JOLIVET, Michel, F-69720 Saint Bonnet de Mure (FR); LETOURNEUR, Odile, F-69110 Sainte Foy les Lyon (FR); NOGUEIRA, Maria, Léonor, F-31000 Toulouse (FR); SEBBAG, Mireille, F-31500 Toulouse (FR); SERRE, Guy, F-31100 Toulouse (FR); VINCENT, Christian, F-31650 Lauzerville (FR)
(74) Mandataire: Guerre, Dominique
(86) Numéro de dépôt international: PCT/FR2002/002032
(87) Numéro de publication internationale: WO 2002/101390

(56) Documents cités:
- EP-A- 0 511 116
- WO-A-98/08946

### LISTE DE SEQUENCES

<110> bioMérieux
<120> PROCEDE DE DETECTION D'AUTO-ANTICORPS SPECIFIQUES DE LA POLYARTHRITE RHUMATOÎDE
<130> PRdelta
<140>
   <141>
<150> FR 01 08068
   <151> 2001-06-13
<160> 6
<170> PatentIn Ver. 2.1
<210> 1
   <211> 399
   <212> PRT
   <213> filaggrine recombinante de rat
<400> 1
   Met Arg Gly Ser His His His His His His Gly Ser Val Asp Glu Ser
    1               5                  10                  15
   Met Gly Ile Gln Gln Gln Arg Gln Arg Arg Gln His Glu Gln Glu Arg
               20                  25                  30
   Glu His Glu His Gln Gln Pro Glu Ser Ser His Arg Gln Gln Gly Ser
           35                  40                  45
   Ser Gly Arg Thr His Arg Ala Ala Arg His Glu Gln Glu Ser Asp Ser
       50                  55                  60
   Thr Arg Gln Arg Gly Ser His Gln Ala His Ser Ser Ala Arg Thr Gln
   65                  70                  75                  80
   Glu Glu Ile Ala Arg Gly Arg Ser Gly Ala Ser Ala Ser Glu Gly Pro
                   85                  90                  95
   Gly Pro Gln Arg Glu Ala Ala Arg Asp Ser Ser Glu His Ala Gln Ser
               100                 105                 110
   Arg Arg Ser Glu Thr Ile Ser Arg Gly Arg Ser Gly His Ser Thr Gly
           115                 120                 125
   Arg Ala His Glu Asp Arg His Glu Gln Ala Thr Asp Arg Ser Ala Arg
       130                 135                 140
   Ser Gly Ser Arg Gly Gly Gln Ala Gly Ser His Ser Glu Ser Glu Ala
   145                 150                 155                 160
   Ser Gly Gly Gln Ala Gly Arg Arg Gly Thr Ala Ala Thr Arg His Thr
                   165                 170                 175
   Ser Arg Pro Glu Gln Ser Pro Asp Thr Ala Gly Arg Thr Gly Ser Ser
               180                 185                 190
   Arg Gly Gln Gln Ser Ala Gln Arg His Ala Asp Ser Thr Pro Gly Ser
   195 200 205
   Thr Arg Thr Gly Ser Arg Gly Arg Gly Glu Ser Pro Ala Gly Gln Gln
   210 215 220
   Ser Pro Asp Arg Ala Arg His Ile Glu Ser Arg Arg Gly Arg Thr Arg
   225 230 235 240
   Glu Ala Ser Ala Ser Gln Ser Ser Asp Ser Glu Gly His Ser Gly Ala
   245 250 255
   His Ala Gly Ile Gly Gln Gly Gln Thr Ser Thr Thr His Arg Arg Ala
   260 265 270
   Gly Ser Ser Ser Gly Ser Gln Arg Ala Ser Ala Gly Gly Gln Ala Ala
   275 280 285
   Asp Thr Ser Ser Arg Ser Gly Ala Ser Gln Gly Gln Ala Ser Gly Gln
   290 295 300
   Gly Arg Ala Gly Ser Arg Ser Arg Glu Ala Gln Gly Ile Ala Arg His
   305 310 315 320
   Gly Gln Ser Thr Asp Ser His Arg His Ser Gly Ala Gly Gln Ala Gln
   325 330 335
   Ala His Thr Ala Thr Ser Ile Asp Ser Arg Arg Pro Arg Gly Ser Ser
   340 345 350
   Glu Ser Gln Ala Ser Asp Ser Glu Gly His Ser Asp Tyr Ser Glu Ala
   355 360 365
   His Thr Gln Gly Ala His Gly Gly Ile Gln Thr Ser Gln Arg His Glu
   370 375 380
   Gln Arg Pro Ser Arg Gly Gln Gln Gly Ser Gly His Pro Gln Val
   385 390 395
<210> 2
   <211> 49
   <212> PRT
   <213> peptide de la filaggrine humaine
<400> 2
   Ser Thr Gly His Ser Gly Ser Gln His Ser His Thr Thr Thr Gln Gly
   1 5 10 15
   Arg Ser Asp Ala Ser Arg Gly Ser Ser Gly Ser Arg Ser Thr Ser Arg
   20 25 30
   Glu Thr Arg Asp Gln Glu Gln Ser Gly Asp Gly Ser Arg His Ser Gly
   35 40 45
   Ser
<210> 3
   <211> 14
   <212> PRT
   <213> peptide de la filaggrine humaine
<400> 3
   Glu Gln Ser Ala Asp Ser Ser Arg His Ser Gly Ser Gly His
    1               5                  10
<210> 4
   <211> 14
   <212> PRT
   <213> peptide de la filaggrine humaine
<400> 4
   Glu Ser Ser Arg Asp Gly Ser Arg His Pro Arg Ser His Asp
    1               5                  10
<210> 5
<211> 24
   <212> PRT
   <213> peptide de la filaggrine de rat
<400> 5
   Gly Gln Ala Gly Gly Arg Gln Gly Ser Arg His Glu Gln Gly Ser Ser
    1               5                  10                  15
   Arg Gly Arg Ser Gly His Glu Gln
                20
<210> 6
   <211> 28
   <212> PRT
   <213> peptide de la filaggrine de rat
<400> 6
   Gly Ser His Arg Gln Gln Ser Ser Arg Arg Gln Gly Ser Ser Arg Gly
    1               5                  10                  15
   Gln Gln Ser Gly Gly Arg Gln Gly Ser Arg His Glu
                20                  25

## Description

La polyarthrite rhumatoïde (PR) est le plus fréquent des rhumatismes inflammatoires chroniques. Il s'agit d'une maladie auto-immune systémique caractérisée par une inflammation des articulations qui affecte plus de 500 000 personnes en France et plus de 2 millions de personnes aux Etats-Unis. Le sérum des patients atteints contient des auto-anticorps dont certains sont spécifiques et peuvent constituer un marqueur de la maladie permettant son diagnostic même à un stade précoce.

Des auto-anticorps spécifiquement présents chez les malades atteints de PR et réagissant avec un antigène épithélial oesophagien de rat ont été décrits pour la première fois par B. J. J. Young et al. dans Br. Med. J. 2 :97-99 (1979). A l'époque ces auto-anticorps ont été dénommés « anticorps anti-kératines » (AKA), mais il a par la suite été montré que ces anticorps étaient en fait des auto-anticorps anti-filaggrine (AAF). Leurs antigènes sont les formes acido-neutres des filaggrines (Simon et al., J. Clin. Invest., 92, 1387, (1993)).

La filaggrine est générée à partir de la profilaggrine, qui est une polyprotéine phosphorylée des granules kératohyalins d'épiderme. La profilaggrine a un poids moléculaire élevé (environ 400 000 chez l'homme), elle est soluble en présence de fortes concentrations de sels ou d'urée. Elle possède une forte teneur en acides aminés basiques (arginine et histidine), ainsi qu'en glycine, sérine et acide glutamique. Elle est pauvre en acides aminés non polaires et ne contient ni méthionine, ni cystéine, ni tryptophane. Elle est fortement phosphorylée sur des résidus sérine, ce qui lui confère un point isoélectrique proche de la neutralité.

La profilaggrine est clivée en unités filaggrine au cours d'un processus complexe de maturation, impliquant une déphosphorylation, suivie d'un clivage par des protéases au niveau des segments interdomaines. Ce clivage génère d'abord des fragments de taille intermédiaire, puis les molécules fonctionnelles de filaggrine.

Les filaggrines issues de la déphosphorylation et du clivage de la profilaggrine sont des protéines basiques dont le contenu en acides aminés est similaire à celui des profilaggrines. Elles participent à l'organisation des filaments de kératine, et subissent une maturation progressive au cours de laquelle les résidus arginine, basiques, sont convertis en résidus citrulline, neutres, sous l'action de la peptidylarginine déiminase (Harding C.R. et Scott I.R., J. Mol. Biol. 170, p. 651-673 (1983)). Ceci entraîne une réduction de leur affinité pour les kératines dont elles se détachent ; elles sont alors totalement dégradées sous l'action de diverses protéases.

Les propriétés des filaggrines et des profilaggrines ont été particulièrement bien étudiées chez le rat, chez la souris et chez l'homme. La taille de la profilaggrine varie, selon les espèces, de 300 à 400 kD et celle des filaggrines de 27 à 64 kD.

Le gène codant pour la profilaggrine se compose de sous-unités répétées dont chacune code pour une molécule de filaggrine, séparées par des portions codant pour les segments peptidiques interdomaines. Toutes les unités de répétition codant pour chacune des filaggrines humaines ont la même longueur (972 paires de base chez l'homme) ; cependant, chez l'homme, on observe des variations importantes (10-15%) de séquence d'une sous-unité à l'autre. Si la plupart sont conservatives, certaines de ces variations induisent des changements d'acides aminés et dans certains cas des changements de la charge électrique de la protéine. Ainsi les filaggrines humaines forment, indépendamment des modifications post-transcriptionnelles, une population hétérogène de molécules de taille similaire mais de séquences et de charges (pHi égal à 8,3 ± 1,1) différentes (Gan et al., Biochem. 29, p. 9432-9440 (1990)).

Le polymorphisme observé chez l'homme entre les séquences des unités filaggrine à l'intérieur d'un même gène de profilaggrine n'apparaît pas chez le rat et la souris. Les filaggrines présentent en outre une grande variabilité inter et intra-spécifique au niveau de leur séquence. Cette variabilité n'affecte toutefois pas leurs propriétés fonctionnelles, ni leur composition globale en acides aminés, et leurs propriétés biochimiques. De même, les localisations tissulaires de la profilaggrine et des filaggrines sont identiques chez les différents mammifères étudiés.

Les auto-anticorps (AAF) sont liés aux paramètres cliniques et biologiques qui définissent les formes les plus actives et les plus sévères de la maladie, et précéderaient l'apparition des signes cliniques. Leur détection s'avère donc une étape essentielle dans le diagnostic de la PR.

Selon le document WO-A-98/08946 au nom de la Demanderesse, on a mis au point un procédé de détection des AAF, dans un échantillon biologique, basé sur la découverte selon laquelle la citrullination de la filaggrine était nécessaire à la détection des AAF présents dans des sérums de patients atteints de PR, cette citrullination générant des épitopes reconnus par les AAF. Ce procédé comprend les étapes suivantes :
on met en contact l'échantillon biologique analysé avec une filaggrine recombinante citrullinée ou un fragment de celle-ci, dans des conditions appropriées pour la formation d'immuno-complexes avec les AAF, et
on détecte les immuno-complexes éventuellement formés avec les AAF.

Les résultats mettent en évidence une forte sensibilité du procédé, une majorité des sérums de patients atteints de PR reconnaissant la filaggrine recombinante citrullinée.

On s'est cependant aperçu, au cours des étapes du diagnostic de la PR chez les patients dont les sérums se révélaient positifs dans ce procédé, que tous n'étaient pas des cas PR. Ce procédé conduit en effet à la détection de faux positifs, comme cela sera illustré dans les exemples qui suivront, et ne peut donc être utilisé de manière fiable, en raison d'une spécificité insuffisante.

Les présents inventeurs ont maintenant mis au point un nouveau test ELISA pour le diagnostic de la PR chez des patients, qui possède une grande spécificité tout en conservant la forte sensibilité du procédé précité.

Selon ce procédé, on détecte des auto-anticorps (AAF) spécifiques de la polyarthrite rhumatoïde (PR), dans un échantillon biologique susceptible de contenir lesdits auto-anticorps (AAF) et d'autres anticorps non spécifiques de la PR, comme suit :
on dispose, d'une part, d'une filaggrine (FNC) ou d'un dérivé de la filaggrine (FNC) ou d'un peptide de la filaggrine (PFNC) ledit peptide comportant après citrullination, un épitope de la filaggrine, et d'autre part, de ladite filaggrine citrullinée (FC) ou d'un dérivé de ladite filaggrine citrullinée (FC) ou dudit peptide citrulliné (PFC), la citrullination consistant en la conversion d'un ou de résidus arginine en citrulline,
on met ledit échantillon biologique en contact avec, d'une part, ladite filaggrine (FNC) ou ledit dérivé de ladite filaggrine (FNC) ou ledit peptide (PFNC), et d'autre part, ladite filaggrine (FC) ou ledit dérivé de ladite filaggrine (FC) ou ledit peptide (PFC), dans des conditions appropriées pour la formation d'immuno-complexes avec les auto-anticorps (AAF),
on détecte et on quantifie les immuno-complexes formés entre les auto-anticorps (AAF) ou les autres anticorps présents dans l'échantillon et ladite filaggrine (FNC) ou ledit dérivé de ladite filaggrine (FNC) ou ledit peptide (PFNC), d'une part, et ladite filaggrine citrullinée (FC) ou ledit dérivé de ladite filaggrine citrullinée (FC) ou ledit peptide (PFC), d'autre part, cette quantification étant exprimée par une valeur respectivement X_{NC} et X_{C},
on retranche la valeur de X_{NC} à celle de X_{C}.

Pour la mise en oeuvre du procédé de l'invention la filaggrine (FNC) ou le dérivé d'une filaggrine (FNC) ou le peptide (PFNC) est immobilisé sur un support solide et la filaggrine (FC) ou un dérivé de la filaggrine (FC) ou le peptide (PFC) est immobilisée sur un autre support solide. Il peut s'agir, entre autres, de deux puits d'une plaque de microtitration ou de deux cônes de l'appareil Vidas (marque enregistrée) commercialisé par la Demanderesse, mais également de bandelettes ou tout autre support approprié.

Avant de décrire l'invention plus en détails et d'en présenter les variantes préférentielles, quelques termes employés dans la description et les revendications sont ci-après définis.

La citrullination est la réaction de conversion d'un résidu arginine en citrulline par transformation du groupe -C(NH)NH₂ de l'arginine en -CONH₂. La réaction est avantageusement effectuée *in vitro* par l'enzyme peptidyl arginine déiminase (PAD). Cette enzyme peut être une PAD de muscle de lapin ; il est cependant à la portée de l'homme du métier de sélectionner une autre PAD appropriée. On peut bien évidemment envisager tout autre moyen de citrullination, tel que des réactifs chimiques, des microorganismes.

Selon l'invention, lorsqu'il est fait référence à une filaggrine citrullinée, on entend qu'une proportion d'environ au moins 20% des résidus arginine ont été convertis en résidus citrulline, cette conversion étant effectuée au hasard. De préférence, ce taux est d'au moins 30%, et avantageusement d'au moins 50%. Il a été observé que l'immunoréactivité d'une filaggrine citrullinée dont le taux de citrullination est d'environ 80% est similaire à celle d'une filaggrine dont le taux de citrullination est d'environ 50%. Dans ces conditions, il n'est donc pas utile de chercher à atteindre des taux très élevés de citrullination.

Si la citrullination n'est pas faite au hasard, mais est régiospécifique, et en particulier, si l'on peut limiter la réaction de conversion à un résidu arginine appartenant à un épitope de la filaggrine après citrullination, le taux de citrullination peut être plus faible que les valeurs précitées, sans que l'immunoréactivité de la filaggrine ainsi citrullinée en soit affectée.

Par dérivé d'une filaggrine (FNC) ou dérivé citrulliné correspondant on entend : une filaggrine chimère qui consiste en une association de fragment(s) de séquences de filaggrine humaine et de rat et/ou une association de fragment(s) de séquences de filaggrine humaine et/ou de rat et de peptide(s) consensus.

Pour la mise en oeuvre du procédé de l'invention, la filaggrine est de préférence choisie parmi la filaggrine humaine et une filaggrine d'origine animale. Elle consiste avantageusement en une filaggrine recombinante de rat, ayant la séquence SEQ ID NO :1.

A la place de la filaggrine, on peut choisir un peptide de la filaggrine (PFNC), dont la séquence comprend au moins un résidu arginine, et le peptide correspondant citrulliné (PFC) pour lequel ledit résidu arginine au moins de PFNC aura été converti en résidu citrulline. Selon une variante du procédé de l'invention, le peptide comporte un épitope de la filaggrine après citrullination. Ainsi, un peptide (PFNC) peut être notamment choisi parmi les peptides suivants :
■ le peptide S47S dont la séquence est représentée en SEQ ID NO : 2 correspondant aux acides aminés 71 à 119 de la séquence d'une unité de filaggrine humaine et comportant 6 résidus arginine,
■ le peptide E12H dont la séquence est représentée en SEQ ID NO : 3 telle que déterminée par référence aux séquences nucléotidiques du gène de la profilaggrine humaine décrites par Gan S.Q et al. (Biochemistry, 29 : 9432-9440, (1990)) et comprenant 1 résidu arginine,
■ le peptide E12D dont la séquence est représentée en SEQ ID NO : 4 telle que déterminée par référence aux séquences nucléotidiques du gène de la profilaggrine humaine décrites par Gan S.Q et al. (Biochemistry, 29 : 9432-9440, (1990)) et comprenant 3 résidus arginine,
■ le peptide G22Q dont la séquence est représentée en SEQ ID NO : 5 correspondant à une séquence consensus d'une unité de filaggrine de rat comprenant 4 résidus arginine,
■ le peptide G26E dont la séquence est représentée en SEQ ID NO : 6 correspondant à une séquence consensus d'une unité de filaggrine de rat comprenant 10 résidus arginine.

Comme dit précédemment, si l'on dispose d'une filaggrine (FNC) pour mettre en oeuvre le procédé de l'invention, la filaggrine citrullinée (FC) utilisée présente la séquence peptidique de la filaggrine (FNC) dans laquelle au moins 20%, de préférence au moins 30%, et mieux encore au moins 50% des résidus arginine de ladite FNC sont citrullinés.

Les auto-anticorps (AAF) sont circulants, et un échantillon biologique à analyser selon le procédé de l'invention peut être choisi parmi le sang, le plasma, le sérum.

La détection et la quantification des immuno-complexes formés sont réalisées par toutes techniques bien connues de l'homme du métier, et en particulier par l'utilisation d'une enzyme pour la détection et de son substrat colorimétrique ou fluorescent pour la quantification (exemples : phosphatase alcaline / 4-méthylumbelliferyl phosphate, peroxydase / orthophénylène diamine).

Comme cela sera illustré dans les exemples, on peut ainsi mettre en contact les immuno-complexes formés avec un conjugué constitué par un anticorps marqué dirigé contre les immunoglobulines humaines, dans des conditions appropriées pour la formation d'immuno-complexes marqués, puis on détecte et on quantifie les immuno-complexes marqués. Le conjugué est avantageusement un anticorps anti-immunoglobuline humaine, entier ou fragmenté (exemples : fragments Fab', Fab'₂), marqué à la peroxydase ou à la phosphatase alcaline, puis on détecte et on quantifie la formation d'immuno-complexes marqués par colorimétrie ou fluorimétrie, la quantification étant exprimée en valeurs X_{NC} et X_{C} de densité optique ou de fluorescence.

Un test positif révélant la présence d'auto-anticorps (AAF) spécifiques de la PR, dans l'échantillon analysé, est caractérisé par la valeur X_{C} supérieure à la valeur X_{NC}.

L'invention est ci-après illustrée et ses caractéristiques et avantages mis en lumière.

### Exemple 1 : Obtention d'une filaggrine recombinante de rat

### A) Clonage du gène de la profilaggrine du rat

Les travaux de Haydock et Dale, 1986 (J. Biol. Chem., 261, 12520-12525) et de Rothnagel et al.,1987 (J. Biol. Chem., 262, 15643-15648) ont montré que le gène de la profilaggrine de rat était composé de 20 ± 2 unités répétées d'environ 1200 paires de bases (pb) et ne disposait pas d'intron dans sa région codante, chaque unité de profilaggrine étant composée d'un domaine filaggrine et d'une séquence de liaison.

D'autre part, la séquence codante pour deux unités de profilaggrine incomplètes a été publiée par Haydock et Dale, 1990 (DNA Cell Biol.,9, 251-261).

A partir de ces données, on a conçu des oligonucléotides destinés à l'amplification par PCR de la séquence codante pour une unité profilaggrine de rat complète. La matrice ADN génomique de rat a été préparée à partir de sang total d'un rat Wistar. Trois produits de PCR différents ont été obtenus avec trois paires d'oligonucléotides différents. Ces produits de PCR ont été clonés dans un vecteur pCAPs (PCR cloning kit, Boehringer) et séquencés. Ils présentent entre eux un degré d'homologie élevé (de plus de 99%) ainsi qu'une homologie de 95 et 98% avec les deux unités de profilaggrine incomplètes publiées par Haydock et Dale, 1990.

Ces données confirment la nature de l'ADN obtenu et suggèrent que les séquences disponibles sont représentatives des domaines filaggrine de rat existant, une certaine variabilité inter et intra individu étant attendue.

Une des séquences obtenues a été clonée dans un vecteur d'expression pMR (Cheynet et al.,1993, Prot. Exp. Purif., 4, 367-372) en fusion avec une séquence codante pour 6 histidine en 5'. La construction identifiée sous l'appellation pOL041 a été utilisée pour transformer des bactéries *E. coli .* Elle code pour une protéine de 399 acides aminés et de poids moléculaire théorique de 42 210 présentant la séquence SEQ ID NO:1.

### B) Expression et purification de la filaggrine recombinante de rat

### Culture :

Pour la mise en culture de la souche *E. coli* transformée (BL21 ou DH5α), un isolement sur milieu gélosé avec ampicilline 0,1 mg/ml est réalisé à partir de la banque conservée à -80°C.

A partir d'une colonie isolée, une pré-culture est effectuée en milieu liquide (extrait de levure, glucose 2% et ampicilline 0,1 mg/ml) en Erlens, placés à +37°C et sous agitation orbitale (280 tours/min).

Cette pré-culture permet ensuite l'ensemencement d'Erlens de culture, de 5 I, contenant chacun 1 I du même milieu complété éventuellement par du PMSF 0,5 mM en final. Ces Erlens sont cultivés à +37°C sous agitation à 280 tours/min, jusqu'à l'obtention d'une DO à 600 nm de 0,8. A partir de cette valeur de DO, la culture est poursuivie sur une durée de 3 heures.

La culture est alors arrêtée et centrifugée 25 minutes à 6000g. Les culots bactériens sont alors collectés et conservés à -25°C.

### Purification :

La lyse du culot bactérien est réalisée par action du lyzozyme en présence d'inhibiteur de protéases dans un tampon NaH₂PO₄ 0,1M / NaCl 0,6M / Tween 0,2 % / Azide Na 1mM / Imidazole 6mM pH 7,3 et par sonication. Ce lysat est alors clarifié 30 minutes à 23700g et le surnageant conservé.

La filaggrine recombinante est purifiée par chromatographie par chélation de métaux (Nickel) à +4°C.

Brièvement après rééquilibrage du gel en tampon de lyse, le surnageant de lyse est injecté sur la colonne suivi d'un lavage (et du signal de détection UV 280 nm), par le tampon NaH₂PO₄ 0,1M / NaCl 0,6M /Azide Na 1mM / Imidazole 6mM pH 7,3, jusqu'au retour du signal de détection UV 280 nm à la ligne de base.

La filaggrine recombinante (rFlg), adsorbée sur le gel, est éluée, par le tampon NaH₂PO₄ 0,1 M / NaCl 0,6M / Azide Na 1 mM / Imidazole 0,5M pH 7,15 .

La fraction ainsi obtenue est concentrée sur membrane de 10 kDa.

La dernière étape de purification est effectuée par chromatographie de filtration sur gel sur un support Superdex 200, en tampon Tris 20mM / NaCl 0,3M pH 11. Le concentrat est injecté sur la colonne, les fractions étant collectées par le suivi du signal UV à 280 nm et 214 nm. Cette étape permet d'isoler et de récupérer la filaggrine recombinante d'intérêt, immunologiquement active, dans le second pic d'élution (PM ≈ 42 000).

### Exemple 2 : Citrullination de la filaggrine recombinante

Après dosage des protéines totales par la méthode de Bradford (réactifs Biorad), la citrullination est effectuée, par action de la Peptidyl Arginine Déiminase 4 heures, à +37°C, sous agitation magnétique à raison de 4 Unités enzymatiques / mg de protéines totales, en présence d'EDTA 0,1 M / DTT 1 M / CaCl₂ 0,5 M. Le taux de protéines totales est alors redosé sur la fraction déiminée.

La filaggrine citrullinée est purifée par chromatographie Ni-NTA, puis chromatographie par filtration sur gel.

Le taux de citrullination détecté est de 53%.

Les exemples 3 et 4 suivants illustrent le diagnostic de la PR dans un procédé de détection de l'art antérieur et dans un procédé de détection selon l'invention, pour en permettre la comparaison

Le procédé de détection de l'art antérieur consiste en un test ELISA, du type de celui décrit dans WO-98/08946 au nom de la Demanderesse, mettant en oeuvre de la filaggrine citrullinée, et basé sur la détection des immuno-complexes formés entre les anticorps AAF éventuellement présents dans l'échantillon et la filaggrine citrullinée.

Le procédé de détection selon l'invention est un test ELISA mettant en oeuvre de la filaggrine citrullinée et de la filaggrine non citrullinée et qui est basé sur la détection parallèle des immuno-complexes formés avec la filaggrine citrullinée d'une part et la filaggrine non citrullinée d'autre part, et sur la différence de valeurs entre les deux.

### Exemple 3 :

### A) Echantillons

Les tests ont été effectués sur les échantillons suivants :
63 sérums témoins atteints des pathologies suivantes : algodystrophie, goutte, hémopathie maligne, maladie de Paget, névralgie, pathologie mécanique, périartérite noueuse, polymyosite, rhumatisme lupique, rhumatisme psoriasique, sclérodermie, spondylarthrite ankylosante, spondylarthropathie, syndrome de Goujerot, syndrome de Sharp, vascularite.
65 sérums de patients atteints de polyarthrite rhumatoïde diagnostiquée cliniquement.

### B) Matériel

Plaque Nunc Maxisorp 468667
Etuve à 35-39°C
Laveur Axia microwasher U4402
Lecteur de plaques Biotek (marque de commerce)

### C) Protocole

Dépôt dans les puits : 100 *µ*l par puits de 2,5 *µ*g/ml de filaggrine, pendant 2 heures à 37°C en tampon PBS pH 7,2
3 lavages avec 300 *µ*l par puits de tampon PBS Tween 0,05% pH 7,2
Passivation 1 heure à 37°C avec 250 *µ*l par puits de tampon PBS BSA 1 % pH 7,2
3 lavages avec 300 *µ*l par puits de tampon PBS Tween 0.05% pH 7,2
Incubation pendant 1 heure à 37°C avec 100 *µ*l par puits de sérum dilué au 1/100^{ème} en tampon Tris 10mM, NaCl 350mM, TritonX100 1 %, BSA 1 %, sérum de lapin 10% pH 7,6
3 lavages avec 300 *µ*l par puits de tampon PBS Tween 0.05% pH 7,2
Incubation d'un conjugué anti-IgG humaine dilué en tampon Tris 10mM, NaCl 350mM, TritonX100 1%, BSA 1%, sérum de lapin 10% pH 7.6 ; 100 *µ*l par puits
3 lavages avec 300 *µ*l par puits de tampon PBS Tween 0.05% pH 7,2

### Révélation enzymatique :

- 100 *µ*l d'orthophénylène diamine (OPD) par puits
- incubation 10 min à 18-25°C
- blocage par 100 *µ*l H₂SO₄
- lecture à 492 nm

Les échantillons sont dosés en parallèle sur une plaque revêtue de filaggrine non citrullinée et sur une autre plaque revêtue de filaggrine citrullinée.

Les échantillons sont dosés en simple.

Les résultats sont exprimés en densité optique. Les signaux indiqués à une densité optique de 3 sont égaux ou supérieurs à 3.

### D) Résultats

Les résultats sont rassemblés dans les tableaux 1 et 2 ci-après.

Avec les résultats obtenus sur filaggrine citrullinée selon WO-A-98/08946 (tableau 1), les performances sont les suivantes :

| | | |
|---|---|---|
| 100% spécificité | seuil à 1,337 | 34% sensibilité (22/65) |
| 98% spécificité | seuil à 0,937 | 38% sensibilité (25/65) |
| 95% spécificité | seuil à 0,335 | 61,5% sensibilité (40/65) |

Avec les résultats obtenus sur filaggrine citrullinée - filaggrine non citrullinée selon l'invention (tableau 2), les performances sont les suivantes :

| | | |
|---|---|---|
| 100% spécificité | seuil à 0,731 | 38% sensibilité (25/65) |
| 98% spécificité | seuil à 0,009 | 69% sensibilité (45/65) |
| 95% spécificité | seuil à -0,004 | 77% sensibilité (50/65) |

Dans les deux cas, à 95% de spécificité 3 sérums sont des faux positifs.

Tous les échantillons détectés sur la phase filaggrine citrullinée sont détectés avec le système différentiel à 100% et 98% de spécificité. Un seul sérum est détecté en plus avec le système différentiel. A 95% de spécificité, 7 échantillons sont détectés en plus avec le système différentiel.

Le système différentiel est plus performant que la phase filaggrine citrullinée sur cette population avec 69% de sensibilité pour 98% de spécificité.

Pour cette population, les performances des tests habituellement utilisés sont les suivantes :
GIFOR (immunofluorescence sur coupe d'oesophage de rat)

| | | |
|---|---|---|
| 98% spécificité | seuil à 2 | 49% sensibilité (32/65) |

Gblot

| | | |
|---|---|---|
| 98% spécificité | seuil à 2,25 | 58% sensibilité (38/65) |

Les performances obtenues en ELISA sur filaggrine de rat sont meilleures que les techniques actuellement utilisées, GIFOR et GBlot.

**Tableau 1**

| Résultats en DO à 492nm des tests ELISA sur filaggrine citrullinée (FC) | | | |
|---|---|---|---|
| **Témoins négatifs** | **FC** | **Sérums PR** | **FC** |
| **58** | 0.050 | **78** | 0.051 |
| **57** | 0.056 | **76** | 0.059 |
| **10** | 0.058 | **72** | 0.077 |
| **33** | 0.060 | **74** | 0.077 |
| **51** | 0.062 | **81** | 0.079 |
| **53** | 0.062 | **79** | 0.088 |
| **44** | 0.064 | **73** | 0.091 |
| **54** | 0.066 | **71** | 0.098 |
| **47** | 0.069 | **80** | 0.102 |
| **49** | 0.069 | **75** | 0.109 |
| **38** | 0.070 | **65** | 0.113 |
| **39** | 0.070 | **86** | 0.121 |
| **40** | 0.071 | **66** | 0.124 |
| **50** | 0.072 | **83** | 0.132 |
| **29** | 0.073 | **69** | 0.141 |
| **45** | 0.073 | **77** | 0.147 |
| **25** | 0.076 | **84** | 0.151 |
| **55** | 0.076 | **85** | 0.156 |
| **28** | 0.079 | **90** | 0.157 |
| **42** | 0.083 | **67** | 0.158 |
| **46** | 0.083 | **68** | 0.158 |
| **60** | 0.085 | **70** | 0.202 |
| **43** | 0.087 | **89** | 0.207 |
| **31** | 0.088 | **88** | 0.276 |
| **34** | 0.088 | **92** | 0.299 |
| **37** | 0.090 | **93** | 0.387 |
| **41** | 0.090 | **87** | 0.409 |
| **16** | 0.092 | **64** | 0.434 |
| **35** | 0.092 | **82** | 0.472 |
| **48** | 0.092 | **95** | 0.496 |
| **26** | 0.093 | **98** | 0.499 |
| **52** | 0.093 | **96** | 0.524 |
| **17** | 0.097 | **94** | 0.535 |
| **61** | 0.098 | **100** | 0.611 |
| **27** | 0.111 | **99** | 0.659 |
| **19** | 0.113 | **91** | 0.667 |
| **20** | 0.113 | **103** | 0.726 |
| **4** | 0.116 | **101** | 0.729 |
| **22** | 0.116 | **102** | 0.801 |
| **36** | 0.123 | **97** | 0.853 |
| **18** | 0.124 | **104** | 0.939 |
| **24** | 0.124 | **105** | 1.203 |
| **14** | 0.128 | **106** | 1.240 |
| **12** | 0.129 | **107** | 1.526 |
| **23** | 0.130 | **108** | 1.694 |
| **7** | 0.133 | **109** | 1.900 |
| **5** | 0.134 | **110** | 2.076 |
| **9** | 0.135 | **111** | 2.387 |
| **30** | 0.142 | **114** | 2.593 |
| **59** | 0.146 | **112** | 2.610 |
| **6** | 0.158 | **113** | 3.000 |
| **21** | 0.159 | **115** | 3.000 |
| **11** | 0.166 | **116** | 3.000 |
| **32** | 0.167 | **117** | 3.000 |
| **15** | 0.169 | **118** | 3.000 |
| **62** | 0.193 | **119** | 3.000 |
| **56** | 0.222 | **120** | 3.000 |
| **3** | 0.253 | **121** | 3.000 |
| **13** | 0.269 | **122** | 3.000 |
| **1** | 0.335 | **123** | 3.000 |
| **8** | 0.369 | **124** | 3.000 |
| **63** | 0.937 | **125** | 3.000 |
| **2** | 1.337 | **126** | 3.000 |
| | | **127** | 3.000 |
| | | **128** | 3.000 |

**Tableau 2**

| Résultats en DO à 492nm des tests ELISA sur filaggrine citrullinée (FC) et non citrullinée (FNC) | | | | | | | |
|---|---|---|---|---|---|---|---|
| **témoins négatifs** | **FNC** | **FC** | **FC - FNC** | **sérums PR** | **FNC** | **FC** | **FC - FNC** |
| **1** | 0.780 | 0.335 | -0.445 | **64** | 0.777 | 0.434 | -0.343 |
| **2** | 1.747 | 1.337 | -0.410 | **65** | 0.202 | 0.113 | -0.089 |
| **3** | 0.433 | 0.253 | -0.180 | **66** | 0.204 | 0.124 | -0.080 |
| **4** | 0.268 | 0.116 | -0.152 | **67** | 0.222 | 0.158 | -0.064 |
| **5** | 0.271 | 0.134 | -0.137 | **68** | 0.221 | 0.158 | -0.063 |
| **6** | 0.294 | 0.158 | -0.136 | **69** | 0.195 | 0.141 | -0.054 |
| **7** | 0.262 | 0.133 | -0.129 | **70** | 0.255 | 0.202 | -0.053 |
| **8** | 0.492 | 0.369 | -0.123 | **71** | 0.131 | 0.098 | -0.033 |
| **9** | 0.231 | 0.135 | -0.096 | **72** | 0.109 | 0.077 | -0.032 |
| **10** | 0.149 | 0.058 | -0.091 | **73** | 0.115 | 0.091 | -0.024 |
| **11** | 0.256 | 0.166 | -0.090 | **74** | 0.099 | 0.077 | -0.022 |
| **12** | 0.218 | 0.129 | -0.089 | **75** | 0.131 | 0.109 | -0.022 |
| **13** | 0.351 | 0.269 | -0.082 | **76** | 0.072 | 0.059 | -0.013 |
| **14** | 0.209 | 0.128 | -0.081 | **77** | 0.157 | 0.147 | -0.010 |
| **15** | 0.248 | 0.169 | -0.079 | **78** | 0.055 | 0.051 | -0.004 |
| **16** | 0.158 | 0.092 | -0.066 | **79** | 0.090 | 0.088 | -0.002 |
| **17** | 0.162 | 0.097 | -0.065 | **80** | 0.104 | 0.102 | -0.002 |
| **18** | 0.188 | 0.124 | -0.064 | **81** | 0.079 | 0.079 | 0.000 |
| **19** | 0.174 | 0.113 | -0.061 | **82** | 0.466 | 0.472 | 0.006 |
| **20** | 0.173 | 0.113 | -0.060 | **83** | 0.124 | 0.132 | 0.008 |
| **21** | 0.219 | 0.159 | -0.060 | **84** | 0.137 | 0.151 | 0.014 |
| **22** | 0.171 | 0.116 | -0.055 | **85** | 0.137 | 0.156 | 0.019 |
| **23** | 0.179 | 0.130 | -0.049 | **86** | 0.101 | 0.121 | 0.020 |
| **24** | 0.172 | 0.124 | -0.048 | **87** | 0.372 | 0.409 | 0.037 |
| **25** | 0.123 | 0.076 | -0.047 | **88** | 0.236 | 0.276 | 0.040 |
| **26** | 0.139 | 0.093 | -0.046 | **89** | 0.137 | 0.207 | 0.070 |
| **27** | 0.153 | 0.111 | -0.042 | **90** | 0.057 | 0.157 | 0.100 |
| **28** | 0.120 | 0.079 | -0.041 | **91** | 0.514 | 0.667 | 0.153 |
| **29** | 0.113 | 0.073 | -0.040 | **92** | 0.058 | 0.299 | 0.241 |
| **30** | 0.181 | 0.142 | -0.039 | **93** | 0.134 | 0.387 | 0.253 |
| **31** | 0.126 | 0.088 | -0.038 | **94** | 0.240 | 0.535 | 0.295 |
| **32** | 0.204 | 0.167 | -0.037 | **95** | 0.156 | 0.496 | 0.340 |
| **33** | 0.096 | 0.060 | -0.036 | **96** | 0.122 | 0.524 | 0.402 |
| **34** | 0.122 | 0.088 | -0.034 | **97** | 0.424 | 0.853 | 0.429 |
| **35** | 0.125 | 0.092 | -0.033 | **98** | 0.063 | 0.499 | 0.436 |
| **36** | 0.156 | 0.123 | -0.033 | **99** | 0.208 | 0.659 | 0.451 |
| **37** | 0.122 | 0.090 | -0.032 | **100** | 0.117 | 0.611 | 0.494 |
| **38** | 0.101 | 0.070 | -0.031 | **101** | 0.207 | 0.729 | 0.522 |
| **39** | 0.101 | 0.070 | -0.031 | **102** | 0.165 | 0.801 | 0.636 |
| **40** | 0.098 | 0.071 | -0.027 | **103** | 0.072 | 0.726 | 0.654 |
| **41** | 0.117 | 0.090 | -0.027 | **104** | 0.142 | 0.939 | 0.797 |
| **42** | 0.110 | 0.083 | -0.027 | **105** | 0.231 | 1.203 | 0.972 |
| **43** | 0.112 | 0.087 | -0.025 | **106** | 0.087 | 1.240 | 1.153 |
| **44** | 0.089 | 0.064 | -0.025 | **107** | 0.118 | 1.526 | 1.408 |
| **45** | 0.097 | 0.073 | -0.024 | **108** | 0.268 | 1.694 | 1.426 |
| **46** | 0.107 | 0.083 | -0.024 | **109** | 0.136 | 1.900 | 1.764 |
| **47** | 0.092 | 0.069 | -0.023 | **110** | 0.095 | 2.076 | 1.981 |
| **48** | 0.113 | 0.092 | -0.021 | **111** | 0.127 | 2.387 | 2.260 |
| **49** | 0.089 | 0.069 | -0.020 | **112** | 0.256 | 2.610 | 2.354 |
| **50** | 0.091 | 0.072 | -0.019 | **113** | 0.555 | 3.000 | 2.445 |
| **51** | 0.081 | 0.062 | -0.019 | **114** | 0.128 | 2.593 | 2.465 |
| **52** | 0.112 | 0.093 | -0.019 | **115** | 0.518 | 3.000 | 2.482 |
| **53** | 0.081 | 0.062 | -0.019 | **116** | 0.338 | 3.000 | 2.662 |
| **54** | 0.083 | 0.066 | -0.017 | **117** | 0.315 | 3.000 | 2.685 |
| **55** | 0.092 | 0.076 | -0.016 | **118** | 0.295 | 3.000 | 2.705 |
| **56** | 0.233 | 0.222 | -0.011 | **119** | 0.224 | 3.000 | 2.776 |
| **57** | 0.065 | 0.056 | -0.009 | **120** | 0.184 | 3.000 | 2.816 |
| **58** | 0.058 | 0.050 | -0.008 | **121** | 0.184 | 3.000 | 2.816 |
| **59** | 0.153 | 0.146 | -0.007 | **122** | 0.181 | 3.000 | 2.819 |
| **60** | 0.089 | 0.085 | -0.004 | **123** | 0.171 | 3.000 | 2.829 |
| **61** | 0.095 | 0.098 | 0.003 | **124** | 0.160 | 3.000 | 2.840 |
| **62** | 0.184 | 0.193 | 0.009 | **125** | 0.145 | 3.000 | 2.855 |
| **63** | 0.206 | 0.937 | 0.731 | **126** | 0.081 | 3.000 | 2.919 |
| | | | | **127** | 0.062 | 3.000 | 2.938 |
| | | | | **128** | 0.056 | 3.000 | 2.944 |

### Exemple 4 :

Cet exemple permet de comparer un test de diagnostic selon l'invention, le test ArFa-ELlSA, et plusieurs tests de diagnostic de l'art antérieur disponibles commercialement ou mettant en oeuvre des méthodes connues, à savoir les tests AKA par immunofluorescence, AhFA-IB, AhFA-ELISA et CCP-ELISA.

### A) Echantillons

Les tests ont été pratiqués sur 711 sérums dont 240 provenaient de patients atteints de polyarthrite rhumatoïde (PR) et 471 provenaient de patients non PR. Parmi les patients non PR, 157 étaient atteints d'une maladie inflammatoire choisie parmi le lupus érythémateux progressif (21), la sclérodermie généralisée (9), le rhumatisme psiorasique (43), la spondylarthrite ankylosante (40) et autres maladies inflammatoires (44), et 314 étaient atteints d'une maladie non inflammatoire choisie parmi l'arthrose (104), la neuropathie compressive (25), la maladie de Paget (68), la dystrophie sympathique réflexe (29), les maladies graves des os (49) et d'autres maladies non inflammatoires (39).

La filaggrine est une filaggrine recombinante de rat ; la filaggrine non citrullinée est obtenue conformément à l'Exemple 1, et la filaggrine citrullinée est obtenue par citrullination (taux de citrullination des résidus arginine de 40%) de la filaggrine non citrullinée conformément à l'Exemple 2.

### B) Protocole

La détection des anticorps selon les tests AKA par immunofluorescence et AhFA-IB a été effectuée selon les méthodes décrites dans C Vincent et al., Ann. Rheum. Dis. (1989) 48, 712-722, et C. Vincent et al., J. Rheumatol. (1998) 25, 838-846.

Le test AhFA-ELlSA est utilisé selon la méthode décrite par L. Nogueira et al., Ann. Rheum. Dis. (2001) 60, 882-887.

Le test CCP-ELISA est réalisé avec le matériel Immunoscan^{™} RA (Euro-diagnostica, Arnhem, Pays-Bas) utilisé selon les recommandations du fabricant.

Le test ArFA-ELlSA selon l'invention a été mis en oeuvre conformément au protocole C) décrit dans l'Exemple 3, à l'exception du fait que dans le présent exemple, les échantillons ont été dosés en quadruple. La variation moyenne entre les essais pour un même échantillon était inférieure à 6%.

Les résultats obtenus pour chacun des tests sont présentés dans le tableau 3 ci-après.

**Tableau 3**

| | **95% specificity** | **98.5% specificity** | **99% specificity** |
|---|---|---|---|
| **'AKA'** | 0.52 (0.45 - 0.58) | 0.45 (0.38 - 0.51) | 0.40 (0.34 - 0.46) |
| **AhFA-IB** | 0.59 (0.53 - 0.65) | 0.48 (0.42 - 0.55) | 0.37 (0.31 - 0.43) |
| **AhFA-ELISA** | 0.53 (0.46 - 0.59) | 0.38 (0.32 - 0.44) | 0.37 (0.31 - 0.43) |
| **CCP-ELISA** | nd | 0.58 (0.52 - 0.65) | 0.50 (0.44 - 0.57) |
| **ArFA-ELISA** | 0.76 (0.70 - 0.81) | 0.67 (0.60 - 0.73) | 0.65 (0.59 - 0.71) |

Il ressort de ce tableau que le test selon l'invention est le plus fiable des tests examinés dans la détection de la PR.

## Revendications

1. Procédé de détection d'auto-anticorps (AAF) spécifiques de la polyarthrite rhumatoïde (PR), dans un échantillon biologique susceptible de contenir lesdits auto-anticorps (AAF) et d'autres anticorps non spécifiques de la PR, selon lequel
on dispose, d'une part, d'une filaggrine (FNC) ou d'un dérivé d'une filaggrine (FNC) ou d'un peptide de la filaggrine (PFNC), ledit peptide comportant, après citrullination, un épitope de la filaggrine, et d'autre part, de ladite filaggrine citrullinée (FC) ou d'un dérivé de ladite filaggrine citrullinée (FC) ou dudit peptide citrulliné (PFC), la citrullination consistant en la conversion d'un ou de résidus arginine en citrulline,
on met ledit échantillon biologique en contact avec, d'une part, ladite filaggrine (FNC) ou ledit dérivé de la filaggrine (FNC) ou ledit peptide (PFNC), et d'autre part, ladite filaggrine citrullinée (FC) ou ledit dérivé de ladite filaggrine citrullinée (FC) ou ledit peptide citrulliné (PFC), dans des conditions appropriées pour la formation d'immuno-complexes avec les auto-anticorps (AAF),
on détecte et on quantifie la formation d'immuno-complexes formés entre les auto-anticorps (AAF) ou d'autres anticorps présents dans l'échantillon et ladite filaggrine (FNC) ou ledit dérivé de la filaggrine (FNC) ou ledit peptide (PFNC), d'une part, et ladite filaggrine citrullinée (FC) ou ledit dérivé de ladite flaggrine citrullinée (FC) ou ledit peptide citrulliné (PFC), d'autre part, cette quantification étant exprimée par une valeur respectivement X_{NC} et X_{C},
on retranche la valeur de X_{NC} à celle de X_{C}.

2. Procédé selon la revendication 1, **caractérisé en ce que** la filaggrine est choisie parmi la filaggrine humaine et la filaggrine de rat.

3. Procédé selon la revendication 2, **caractérisé en ce que** la filaggrine est une flaggrine recombinante de rat et a la séquence SEQ ID NO : 1.

4. Procédé selon la revendication 1, **caractérisé en ce que** le peptide comprend une séquence peptidique choisie parmi SEQ ID NO:2 à SEQID NO : 6.

5. Procédé selon la revendication 1, **caractérisé en ce que** la filaggrine citrullinée (FC) ou le peptide citrulliné (PFC) est obtenu par action de la peptidyl arginine déiminase.

6. Procédé selon les revendications 1 et 5, **caractérisé en ce qu'**on dispose d'une filaggrine (FNC) et **en ce qu'**au moins 20% des résidus arginine de la FNC sont citrullinés pour obtenir ladite filaggrine citrullinée (FC).

7. Procédé selon la revendication 6, **caractérisé en ce qu'**au moins 30%, de préférence au moins 50% des résidus arginine de la FNC sont citrullinés.

8. Procédé selon la revendication 1, **caractérisé en ce que** l'échantillon biologique est choisi parmi le sang, le plasma, le sérum.

9. Procédé selon la revendication 1, **caractérisé en ce qu'**on met en contact les immuno-complexes formés avec un conjugué constitué par un anticorps marqué dirigé contre les immunoglobulines humaines, dans des conditions appropriées pour la formation d'immuno-complexes marqués, puis on détecte et on quantifie les immuno-complexes marqués.

10. Procédé selon la revendication 9, **caractérisé en ce que** le conjugué est un anticorps anti-immunoglobuline humaine marqué à la phosphatase alacaline ou à la peroxydase, puis on détecte et on quantifie la formation d'immuno-complexes marqués par colorimétrie ou fluorimétrie, la quantification étant exprimée en valeurs X_{NC} et X_{C} de densité optique ou de fluorescence.

11. Procédé selon la revendication 1, **caractérisé en ce que** si la valeur X_{C} est supérieure à la valeur X_{NC}, on a détecté et quantifié, dans ledit échantillon, des auto-anticorps (AAF) spécifiques de la PR.

## Claims

1. Method for detecting rheumatoid arthritis (RA)-specific autoantibodies (AFAs) in a biological sample liable to contain said autoantibodies (AFAs) and other antibodies not specific for RA, according to which
firstly, a filaggrin (FNC) or a derivative of a filaggrin (FNC) or a filaggrin peptide (PFNC) said peptide containing, after citrullination, a filaggrin epitope, and, secondly, said citrulline-containing filaggrin (FC) or a derivative of said citrulline-containing filaggrin (FC) or said citrulline-containing peptide (PFC), the citrullination consisting of the conversion of one or more arginine residues to citrulline, are provided,
said biological sample is brought into contact with, firstly, said filaggrin (FNC) or said derivative of filaggrin (FNC) or said peptide (PFNC) and, secondly, said citrulline-containing filaggrin (FC) or said derivative of said citrulline-containing filaggrin (FC) or said citrulline-containing peptide (PFC), under conditions suitable for the formation of immune complexes with the autoantibodies (AFAs),
the formation of immune complexes formed between the autoantibodies (AFAs) or other antibodies present in the sample and, firstly, said filaggrin (FNC) or said derivative of filaggrin (FNC) or said peptide (PFNC) and, secondly, said citrulline-containing filaggrin (FC) or said derivative of said citrulline-containing filaggrin (FC) or said citrulline-containing peptide (PFC), is detected and quantified, this quantification being expressed by a value respectively X_{NC} and X_{C},
the value of X_{C} is reduced by the value of X_{NC}.

2. Method according to Claim 1, **characterized in that** the filaggrin is chosen from human filaggrin and rat filaggrin.

3. Method according to Claim 2, **characterized in that** the filaggrin is a recombinant rat filaggrin and has the sequence SED ID No.1.

4. Method according to Claim 1, **characterized in that** the peptide comprises a peptide sequence chosen from SEQ ID No.2 to SEQ ID No.6.

5. Method according to Claim 1, **characterized in that** the citrulline-containing filaggrin (FC) or the citrulline-containing peptide (PFC) is obtained by the action of peptidylarginine deiminase.

6. Method according to Claims 1 and 5, **characterized in that** a filaggrin (FNC) is provided and **in that** at least 20% of the arginine residues of the FNC are converted to citrulline so as to obtain said citrulline-containing filaggrin (FC).

7. Method according to Claim 6, **characterized in that** at least 30%, preferably at least 50%, of the arginine residues of the FNC are converted to citrulline.

8. Method according to Claim 1, **characterized in that** the biological sample is chosen from blood, plasma and serum.

9. Method according to Claim 1, **characterized in that** the immune complexes formed are brought into contact with a conjugate consisting of a labeled antibody directed against human immunoglobulins, under conditions suitable for the formation of labeled immune complexes, and then the labeled immune complexes are detected and quantified.

10. Method according to Claim 9, **characterized in that** the conjugate is an anti-human immunoglobulin antibody labeled with alkaline phosphatase or with peroxidase, and then the formation of labeled immune complexes is detected and quantified by colorimetry or fluorimetry, the quantification being expressed as optical density or fluorescence values X_{NC} and X_{C}.

11. Method according to Claim 1, **characterized in that**, if the value X_{C} is greater than the value X_{NC}, RA-specific autoantibodies (AFAs) have been detected and quantified in said sample.

## Patentansprüche

1. Verfahren zum Nachweisen von für rheumatoide Polyarthritis (RP) spezifischen Autoantikörpern (AFA) in einer biologischen Probe, die diese Autoantikörper (AFA) sowie andere, nicht für die RP spezifische Antikörper enthalten kann, bei dem man folgendermaßen vorgeht:
man verfügt erstens über ein Filaggrin (NCF) oder über ein Filaggrin (NCF)-Derivat oder über ein Filaggrinpeptid (NCFP), wobei dieses Peptid nach Citrullinierung über einen Epitop des Filaggrins verfügt, sowie zweitens über dieses citrullinierte Filaggrin (CF) oder ein Derivat dieses citrullinisierten Filaggrins (CF) oder dieses citrullinierten Peptids (CFP), wobei die Citrullinierung darin besteht, daß ein oder mehrere Argininrest(e) in Citrullin umgewandelt wurde(n),
man bringt diese biologische Probe erstens mit dem Filaggrin (NCF) oder dem Filaggrin (NCF)-Derivat oder dem Peptid (NCFP) und zweitens dem citrullinierten Filaggrin (CF) oder dem Derivat des citrullinierten Filaggrins (CF) oder dem citrullinierten Peptid (CFP) unter Bedingungen, die sich für die Bildung von Immunkomplexen mit den Autoantikörpern (AFA) eignen, in Kontakt,
man weist die Bildung von Immunkomplexen, die zwischen den Autoantikörpern (AFA) oder anderen Antikörpern, die in der Probe vorliegen, und erstens dem Filaggrin (NCF) oder dem Filaggrin (NCF)-Derivat oder dem Peptid (NCFP) und zweitens dem citrullinierten Filaggrin (CF) oder dem Derivat dieses citrullinierten Filaggrins (CF) oder dem citrullinierten Peptid (CFP) gebildet werden, nach und bestimmt diese quantitativ, wobei die quantitative Bestimmung durch einen Wert X_{CN} bzw. X_{C} ausgedrückt wird,
man subtrahiert den Wert X_{CN} von X_{C}.

2. Verfahren nach Anspruch 1, **dadurch gekennzeichnet, daß** das Filaggrin aus der Reihe Human-Filaggrin und Ratten-Filaggrin stammt.

3. Verfahren nach Anspruch 2, **dadurch gekennzeichnet, daß** es sich bei dem Filaggrin um ein rekombinantes Ratten-Filaggrin mit der Sequenz SEQ ID NR: 1 handelt.

4. Verfahren nach Anspruch 1, **dadurch gekennzeichnet, daß** das Peptid eine Peptidsequenz aus der Reihe SEQ ID NR: 2 bis SEQ ID NR: 6 enthält.

5. Verfahren nach Anspruch 1, **dadurch gekennzeichnet, daß** das citrullinierte Filaggrin (CF) oder das citrullinierte Peptid (CFP) durch Einwirkung der Peptidylarginindeiminase erhalten wird.

6. Verfahren nach den Ansprüchen 1 und 5, **dadurch gekennzeichnet, daß** man über ein Filaggrin (NCF) verfügt und das mindestens 20% der Argininreste des NCF citrulliniert werden, um zu dem citrullinierten Filaggrin (CF) zu gelangen.

7. Verfahren nach Anspruch 6, **dadurch gekennzeichnet, daß** mindestes 30%, vorzugsweise mindestens 50%, der Argininreste des NCF citrulliniert werden.

8. Verfahren nach Anspruch 1, **dadurch gekennzeichnet, daß** die biologische Probe aus der Reihe Blut, Plasma und Serum stammt.

9. Verfahren nach Anspruch 1, **dadurch gekennzeichnet, daß** man die gebildeten Immunkomplexe mit einem Konjugat, das aus einem markierten Antikörper gegen Human-Immunglobuline besteht, unter Bedingungen, die zur Bildung von markierten Immunkomplexen geeignet sind, in Kontakt bringt und man die markierten Immunkomplexe anschließend nachweist und quantitativ bestimmt.

10. Verfahren nach Anspruch 9, **dadurch gekennzeichnet, daß** es sich bei dem Konjugat um einen mit alkalischer Phosphatase oder mit Peroxidase markierten Antikörper gegen Human-Immunglobulin handelt und daß man anschließend die Bildung von markierten Immunkomplexen kolorimetrisch oder fluorimetrisch nachweist und quantitativ bestimmt, wobei die quantitative Bestimmung durch die Werte X_{CN} und X_{C} für die optische Dichte oder die Fluoreszenz ausgedrückt wird.

11. Verfahren nach Anspruch 1, **dadurch gekennzeichnet, daß**, wenn der Wert X_{C} größer als der Wert X_{CN} ist, für RP spezifische Autoantikörper (AFA) in dieser Probe nachgewiesen und quantitativ bestimmt worden sind.
